# EUROPÄISCHE PATENTSCHRIFT

(11) **EP 1 377 562 B1**
(45) Veröffentlichungstag und Bekanntmachung des Hinweises auf die Patenterteilung: **09.11.2005**
(21) Anmeldenummer: 02716731.1
(22) Anmeldetag: 06.02.2002
(51) Int. Cl.: C07D 301/12, C07D 303/04

(54) **VERFAHREN ZUR HERSTELLUNG EINES EPOXIDS**
METHOD FOR PRODUCING AN EPOXIDE
PROCEDE POUR PRODUIRE UN EPOXYDE

(30) Priorität: 07.02.2001 DE 10105527
(43) Veröffentlichungstag der Anmeldung: 07.01.2004
(73) Patentinhaber: BASF Aktiengesellschaft, 67056 Ludwigshafen (DE)
(72) Erfinder: TELES, Joaquim, Henrique, 67166 Otterstadt (DE); REHFINGER, Alwin, 67112 Mutterstadt (DE); BERG, Anne, 2170 Merksem (BE); RUDOLF, Peter, 68526 Ladenburg (DE); RIEBER, Norbert, 68259 Mannheim (DE); BASSLER, Peter, 68519 Viernheim (DE)
(74) Vertreter: Isenbruck, Günter
(86) Internationale Anmeldenummer: PCT/EP2002/001218
(87) Internationale Veröffentlichungsnummer: WO 2002/062779

(56) Entgegenhaltungen:
- EP-A- 0 611 761
- EP-A- 0 822 189
- US-A- 3 947 501
- US-A- 5 288 882

## Beschreibung

Die vorliegende Erfindung betrifft ein Verfahren zur Herstellung eines Epoxids (Epoxidierung). Im Rahmen des erfindungsgemäßen Verfahrens wird eine Aufarbeitung des bei der Umsetzung einer organischen Verbindung mit wenigstens einer C-C-Doppelbindung mit Wasserstoffperoxid in Gegenwart wenigstens einer katalytisch aktiven Verbindung und wenigstens eines Lösungsmittels anfallenden Reaktionsgemisches derart vorgenommen, daß die als Nebenprodukt der Epoxidierung entstehenden α-Hydroperoxyalkohole dabei weitgehend unzersetzt bleiben, bzw. zu den jeweils entsprechenden Glykolen umgesetzt werden.

Als Epoxide bezeichnet man Verbindungen, die durch Addition von Sauerstoff an die beiden C-Atome einer Doppelbindung entstanden sind.

In den gängigen Verfahren des Standes der Technik wird die zu epoxidierende Verbindung, in der Regel eine Verbindung mit mindestens einer C-C-Doppelbindung, mit Wasserstoffperoxid in einem Lösungsmittel, beispielsweise Methanol sowie in Gegenwart eines Katalysators in einem entsprechenden Reaktor umgesetzt. Die allgemeinen Prinzipien dieser Umsetzung werden in der DE 198 35 907.1, 100 32 885.7 und 100 15 246.5 beschrieben.

Das gebildete Epoxid wird zusammen mit der nicht umgesetzten zu epoxidierenden Verbindung, Sauerstoff und anderen leichtsiedenden Nebenprodukten sowie einem geringen Teil an Lösungsmittel im Wege der destillativen Aufarbeitung über Kopf abgetrennt. Im Destillationssumpf verbleibt ein Gemisch, welches hauptsächlich aus Lösungsmittel, Wasser, unumgesetztem Wasserstoffperoxid sowie hochsiedenden Nebenprodukten besteht. Dieses kann im Anschluß in einem Nachreaktor erneut mit der zu epoxidierenden Verbindung umgesetzt werden. Als Produktstrom erhält man dabei ein Gemisch, welches sich hauptsächlich aus Lösungsmittel, Wasser, Epoxid und Nebenprodukten zusammensetzt.

Um das gewünschte Epoxid in Reinform zu gewinnen, wird der Kopfstrom der Destillationskolonne sowie der Austrag des Nachreaktors weiter aufgearbeitet.

Die wichtigsten Nebenprodukte, welche bei Epoxidierungen anfallen, sind neben Sauerstoff, Produkte, welche durch die Addition des Lösungsmittels, des Wassers oder des Wasserstoffperoxids an das gebildete Epoxid entstehen können. Als Beispiele sind Alkoxyalkohole, Glykole, sowie α-Hydroperoxyalkohole zu nennen.

Wird als Lösungsmittel beispielsweise Methanol eingesetzt und handelt es sich bei der zu epoxidierenden Verbindung um Propen, so entstehen als wichtigste Nebenprodukte der oben genannten Art 2-Methoxypropanol-1 und 1-Methoxypropanol-2, Propylenglykol, 2-Hydroperoxypropanol-1 und 1-Hydroperoxypropanol-2.

Während Methoxypropanole sowie Propylenglykol sehr stabile Moleküle sind, neigen die Hydroperoxypropanole als thermisch labile Moleküle dazu, sich unter Bildung von weiteren Nebenprodukten, u.a. Formaldehyd, Acetaldehyd, Propylenglykol sowie Hydroxyaceton zu zersetzen. Einige dieser Nebenprodukte neigen wiederum dazu, sich zu weiteren, sekundären Nebenprodukten umzusetzen. Beispielsweise Formaldehyddimethylacetal, Ameisensäure und Methylformiat aus Formaldehyd bzw. Acetaldehyddimethylacetal, Essigsäure und Methylacetat aus Acetaldehyd.

Einige dieser sekundären Nebenprodukte sind es, die bei der weiteren Aufarbeitung des Produktgemisches besonders große Probleme verursachen. Beispielsweise können Substanzen wie Acetaldehyd und Methylformiat nur unter großem Aufwand destillativ aus dem das Epoxid enthaltenden Gemisch abgetrennt werden.

So beschreibt US 6,024,840 ein apparativ aufwendiges Verfahren zur Abtrennung von Acetaldehyd aus Propylenoxid über in Reihe geschaltete Kolonnen zur extraktiven bzw. fraktionierten Destillation.

Aufgrund der dicht beieinanderliegenden Siedepunkte von Methylformiat und Propylenoxid ist bei ihrer Trennung ebenfalls ein hoher apparativer Aufwand notwendig.

So beschreibt US 5,170,002 die Abtrennung von Methylformiat mit Hilfe geeigneter basischer Ionenaustauscherharze, wobei nur das Formiat am Ionenaustauscherharz umgesetzt und somit entfernt wird.

Auch die Rückgewinnung und Wiederaufbereitung von wirtschaftlich wertvollen Substanzen aus dem Produktgemisch und ihr erneuter Einsatz bei der Epoxidierung war bisher aufgrund der Anwesenheit dieser Nebenprodukte mit einem höheren apparativen Aufwand verbunden.

Beispielsweise wird bei Epoxidierungsreaktionen als Lösungsmittel bevorzugt Methanol eingesetzt. Aus wirtschaftlichen Gründen ist man bestrebt, dieses aus dem Produktaustrag zurückzugewinnen, zu reinigen und es dem Reaktionskreislauf wieder zuzuführen. Dabei bereitet insbesondere die Anwesenheit von den im Produktgemisch häufig vorhandenen Nebenprodukten wie Acetaldehyd, Acetaldehyddimethylacetal sowie Methylformiat Probleme. Eine weitgehend vollständige Abtrennung dieser störenden Substanzen konnte bisher nur mit einem hohen apparativen Aufwand bewältigt werden.

So beschreibt US 5,863,391, daß die Abtrennung von Acetaldehyd aus Methanol nur durch extraktive Destillation, durchgeführt in mehreren in Reihe geschalteten Destillationskolonnen, mit einem zufriedenstellenden Ergebnis durchführbar ist. Ebenso aufwendig wird in DE 10032885.7 das Verfahren zur Abtrennung von Methylformiat aus Methanol beschrieben.

Demgemäß lag eine Aufgabe der vorliegenden Erfindung darin, ein Verfahren bereitzustellen, durch welches die Entstehung derart aufwendig aufzuarbeitender Nebenprodukte bei Epoxidierungsreaktionen gänzlich vermieden, bzw. auf ein Minimum reduziert wird und somit auch die oben im Stand der Technik aufgeführten Probleme weitestgehend vermieden werden.

Somit betrifft die vorliegende Erfindung ein Verfahren zur Epoxidierung einer organischen Verbindung mit wenigstens einer C-C-Doppelbindung mit Wasserstoffperoxid in Gegenwart wenigstens einer katalytisch aktiven Verbindung und wenigstens eines Lösungsmittels, dadurch gekennzeichnet, daß ein Produktgemisch umfassend α-Hydroperoxyalkohole unter Einsatz wenigstens eines Reduktionsmittels reduziert wird.

Unter organischen Verbindungen mit mindestens einer C-C- Doppelbindung werden im Rahmen der vorliegenden Erfindung alle organischen Verbindungen verstanden, welche wenigstens eine C=C -Gruppierung aufweisen.

Bevorzugt werden im Rahmen der Erfindung organische Verbindungen der Klasse der Alkene, welche wenigstens eine solche Gruppierung aufweisen, eingesetzt.

Besonders bevorzugt wird im Rahmen des erfindungsgemäßen Verfahrens als Alken Propen eingesetzt.

### Als Beispiele seien folgende Alkene genannt:

Ethen, Propen, 1-Buten, 2-Buten, Isobuten, Butadien, Pentene, Piperylen, Hexene, Hexadiene, Heptene, Octene, Düsobuten, Trimethylpenten, Nonene, Dodecen, Tridecen, Tetra- bis Eicosene, Tri- und Tetrapropen, Polybutadiene, Polyisobutene, Isoprene, Terpene, Geraniol, Linalool, Linalylacetat, Methylencyclopropan, Cyclopenten, Cyclohexen, Norbomen, Cyclohepten, Vinylcyclohexan, Vinyloxiran, Vinylcyclohexen, Styrol, Cycloocten, Cyclooctadien, Vinylnorbornen, Inden, Tetrahydroinden, Methylstyrol, Dicyclopentadien, Divinylbenzol, Cyclododecen, Cyclododecatrien, Stilben, Diphenylbutadien, Vitamin A, Betacarotin, Vinylidenfluorid, Allylhalogenide, Crotylchlorid, Methallylchlorid, Dichlorbuten, Allylalkohol, Methallylalkohol, Butenole, Butendiole, Cyclopentendiole, Pentenole, Octadienole, Tridecenole, ungesättigte Steroide, Ethoxyethen, Isoeugenol, Anethol, ungesättigte Carbonsäuren wie z.B. Acrylsäure, Methacrylsäure, Crotonsäure, Maleinsäure, Vinylessigsäure, ungesättigte Fettsäuren, wie z.B. Ölsäure, Linolsäure, Palmitinsäure, natürlich vorkommende Fette und Öle.

Bevorzugt werden im erfindungsgemäßen Verfahren Alkene verwendet, die 2 bis 18 Kohlenstoffatome enthalten.

Zur Epoxidierung der eben aufgeführten organischen Verbindungen mit mindestens einer C-C-Doppelbindung wird im Rahmen der vorliegenden Erfindung Wasserstoffperoxid eingesetzt.

Zur Herstellung des verwendeten Wasserstoffperoxides kann dabei beispielsweise auf das Anthrachinonverfahren zurückgegriffen werden, nach dem praktisch die gesamte Menge des weltweit produzierten Wasserstoffperoxids hergestellt wird. Dieses Verfahren beruht auf der katalytischen Hydrierung einer Anthrachinon-Verbindung zur entsprechenden Anthrahydrochinon-Verbindung, nachfolgender Umsetzung derselben mit Sauerstoff unter Bildung von Wasserstoffperoxid und anschließender Abtrennung des gebildeten Wasserstoffperoxids durch Extraktion. Der Katalysezyklus wird durch erneute Hydrierung der rückgebildeten Anthrachinon-Verbindung geschlossen.

Einen Überblick über das Anthrachinonverfahren gibt "Ullmanns Encyclopedia of Industrial Chemistry", 5. Auflage, Band 13, Seiten 447 bis 456.

Ebenso ist es denkbar, zur Wasserstoffperoxidgewinnung Schwefelsäure durch anodische Oxidation unter gleichzeitiger kathodischer Wasserstoffentwicklung in Peroxodischwefelsäure zu überführen. Die Hydrolyse der Peroxodischwefelsäure führt dann auf dem Weg über Peroxoschwefelsäure zu Wasserstoffperoxid und Schwefelsäure, die damit zurückgewonnen wird.

Möglich ist selbstverständlich auch die Darstellung von Wasserstoffperoxid aus den Elementen.

Vor dem Einsatz von Wasserstoffperoxid im erfindungsgemäßen Verfahren ist es möglich, beispielsweise eine kommerziell erhältliche Wasserstoffperoxid-Lösung von unerwünschten Ionen zu befreien. Dabei sind unter anderem Methoden denkbar, wie sie beispielsweise in der WO 98/54086, in der DE-A 42 22 109 oder in der WO 92/06918 beschrieben sind. Ebenso kann mindestens ein Salz, das in der Wasserstoffperoxid-Lösung enthalten ist, durch eine Vorrichtung aus der Wasserstoffperoxid-Lösung mittels Ionenaustausch entfernt werden, die dadurch gekennzeichnet ist, daß die Vorrichtung mindestens ein nicht-saures Ionenaustauscherbett mit einer Strömungsquerschnittsfläche F und einer Höhe H aufweist, wobei die Höhe H des Ionenaustauscherbettes kleiner oder gleich 2,5 • F^{1/2} und insbesondere kleiner oder gleich 1,5 • F^{1/2} ist. Im Rahmen der vorliegenden Erfindung können prinzipiell alle nicht-sauren Ionenaustauscherbetten mit Kationenaustauscher und/oder Anionenaustauscher eingesetzt werden. Auch innerhalb eines Ionenaustauscherbettes können Kationen- und Anionenaustauscher als sogenannte Mischbetten verwendet werden. In einer bevorzugten Ausführungsform der vorliegenden Erfindung wird nur ein Typ von nicht-saueren Ionenaustauschern eingesetzt. Weiter bevorzugt ist der Einsatz eines basischen Ionentausches, besonders bevorzugt der eines basischen Anionentauschers und weiter besonders bevorzugt der eines schwach basischen Anionentauschers.

Die erfindungsgemäße Umsetzung der organischen Verbindungen mit mindestens einer C-C-Doppelbindung mit Wasserstoffperoxid findet in Gegenwart wenigstens einer katalytisch aktiven Verbindung statt.

Im allgemeinen eignen sich im Rahmen der vorliegenden Erfindung alle dem Fachmann bekannten Katalysatoren. Bevorzugt werden jedoch Zeolith-Katalysatoren eingesetzt.

Demgemäß betrifft die vorliegende Erfindung auch ein Verfahren der oben genannten Art, dadurch gekennzeichnet daß die wenigstens eine katalytisch aktive Verbindung einen Zeolith-Katalysator umfasst.

Bezüglich der im Rahmen der vorliegenden Erfindung einsetzbaren Zeolith-Katalysatoren existieren keine besonderen Beschränkungen.

Zeolithe sind bekanntermaßen kristalline Alumosilikate mit geordneten Kanal- und Käfigstrukturen, die Mikroporen aufweisen, die vorzugsweise kleiner als ungefähr 0,9 nm sind. Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise bei W. M. Meier, D. H. Olson und Ch. Baerlocher, "Atlas of Zeolithe Structure Types", Elsevier, 4. Aufl., London 1996.

Es sind nun auch Zeolithe bekannt, die kein Aluminium enthalten und bei denen im Silikatgitter an Stelle des Si(IV) teilweise Titan als Ti(IV) steht. Diese Titanzeolithe, insbesondere solche mit einer Kristallstruktur von MFI-Typ, sowie Möglichkeiten zu Ihrer Herstellung sind beschrieben, beispielsweise in der EP-A 0 311 983 oder EP-A 405 978. Außer Silicium und Titan können solche Materialien auch zusätzliche Elemente wie z. B. Aluminium, Zirkonium, Zinn, Eisen, Kobalt, Nickel, Gallium, Bor oder geringe Menge an Fluor enthalten. In den mit dem erfindungsgemäßen Verfahren vorzugsweise regenerierten Zeolith-Katalysatoren kann das Titan des Zeoliths teilweise oder vollständig durch Vanadium, Zirkonium, Chrom oder Niob oder ein Gemisch aus zwei oder mehr davon ersetzt sein. Das molare Verhältnis von Titan und/oder Vanadium, Zirkonium, Chrom oder Niob zur Summe aus Silicium und Titan und/oder Vanadium und/oder Zirkonium, und/oder Chrom und/oder Niob liegt in der Regel im Bereich von 0,01 : 1 bis 0,1 : 1.

Titanzeolithe, insbesondere solche mit einer Kristallstruktur vom MFI-Typ, sowie Möglichkeiten zu ihrer Herstellung sind beispielsweise in der WO 98/55228, WO 98/03394, WO 98/03395, EP-A 0 311 983 oder der EP-A 0 405 978 beschrieben, deren diesbezüglicher Umfang vollumfänglich in den Kontext der vorliegenden Anmeldung einbezogen wird.

Titanzeolithe mit MFI-Struktur sind dafür bekannt, daß sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Dabei sind im einzelnen Titan-, Germanium-, Tellur-, Vanadium-, Chrom-, Niob-, Zirkoniumhaltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen. Denkbar sind für den Einsatz im erfindungsgemäßen Verfahren weiterhin titanhaltige Zeolithe mit der Struktur des ITQ-4, SSZ-24, TTM-1, UTD-1, CIT-1 oder CIT-5. Als weitere titanhaltige Zeolithe sind solche mit der Struktur des ZSM-48 oder ZSM-12 zu nennen.

Bevorzugt werden im Rahmen der vorliegenden Erfindung Ti-Zeolithe mit MFI-, MEL- oder MFI/MEL-Mischstruktur eingesetzt. Als weiterhin bevorzugt sind im einzelnen die Tienthaltenden Zeolith-Katalysatoren, die im allgemeinen als "TS-1", "TS-2", "TS-3" bezeichnet werden, sowie Ti-Zeolithe mit einer zu β-Zeolith isomorphen Gerüststruktur zu nennen.

Demgemäß betrifft die vorliegende Erfindung auch ein Verfahren, wie oben beschrieben, dadurch gekennzeichnet, daß der Katalysator ein Titansilikalit der Struktur TS-1-Struktur ist.

Als Lösungsmittel, einsetzbar bei der Epoxidierung, eignen sich prinzipiell alle dem Fachmann dafür bekannten Lösungsmittel. Bevorzugt werden organische Lösungsmittel, wie Alkohole, einzeln oder als Gemisch aus zwei oder mehr davon, eingesetzt. Auch Alkohol/Wasser Mischungen sind einsetzbar. Bevorzugt wird im Rahmen der vorliegenden Erfindung als Lösungsmittel bei der Epoxidierung Methanol verwendet.

Die dabei einsetzbaren Lösungsmittelmengen sind in breiten Grenzen variierbar. Mögliche Lösungsmittelmengen im Rahmen der Erfindung liegen zwischen 5 und 25 g Methanol pro Gramm eingesetztem Wasserstoffperoxid. Bevorzugt wird das Lösungsmittel in Mengenbereichen von 8 bis 16 g Methanol pro Gramm eingesetztem Wasserstoffperoxid, besonders bevorzugt von 10 bis 14 g Methanol pro Gramm eingesetztem Wasserstoffperoxid, eingesetzt.

Im Rahmen der in Rede stehenden Epoxidierungsreaktion bildet sich ein Produktgemisch, welches u.a. α-Hydroperoxyalkohole aufweist. Dieses Produktgemisch wird reduziert. Die α-Hydroperoxyalkohole werden dabei gezielt zu den entsprechenden Glykolen (1,2-Diole) reduziert, so daß verhindert wird, daß sich die eingangs beschriebenen primären bzw. sekundären Nebenprodukte durch unspezifische Zersetzung bilden können, welche bei der weiteren Aufarbeitung des Produktgemisches die eingangs erwähnten Probleme verursachen. Somit kann die weitere Aufarbeitung des Produktgemisches weitgehend störungsfrei und mit einem, gegenüber dem Stand der Technik reduzierten apparativen Aufwand erfolgen.

Zur Reduktion können alle für diesen Zweck in der Literatur beschriebenen Reduktionsmittel verwendet werden. Vorzugsweise jedoch solche, die in einer wässrigmethanolischen Lösung anwendbar sind. Die Reduktionsmittel können einzeln oder als Gemisch aus zwei oder mehr davon eingesetzt werden.

Im folgenden werden beispielhaft einige in diesem Zusammenhang geeignete Reduktionsmittel bzw. -methoden beschrieben. Dabei können alle dem Fachmann für die jeweilige Reduktion unter Einsatz des jeweilig gewählten Reduktionsmittels bekannten Reduktionsbedingungen gewählt werden. Das jeweilig gewählte Reduktionsverfahren kann kontinuierlich sowie auch diskontinuierlich verlaufen.

So ist es beispielsweise möglich das jeweilige α-Hydroperoxyalkohol-haltige Produktgemisch unter Einsatz von Phosphor(III)-Verbindungen wie PCl₃, Phosphane (z.B.

Triphenylphosphin, Tributylphosphin), Phosphoriger Säure bzw. deren Salze oder Natriumhyphophosphit (NaH₂PO₂) zu reduzieren.

Auch die reduktive Umsetzung mit Schwefel(II)-Verbindungen beispielsweise H₂S oder deren Salze, Natriumpolysulfide (Na₂Sₓ, x>1), Dimethylsulfid, Tetrahydrothiophen, Bis-(hydroxyethyl)-sulfid oder Natriumthiosulfat (Na₂S₂O₃) sowie mit Schwefel(IV)-Verbindungen beispielsweise Schweflige Säure (H₂SO₃) und deren Salze, Natriumbisulfit (Na₂S₂O₅) oder Thioharnstoff-S-oxid führt zum gewünschten Erfolg, der Reduktion von α-Hydroperoxyalkohole zu den entsprechenden Glykolen.

Weiterhin können α-Hydroperoxyalkohole auch durch die Umsetzung des sie enthaltenden Produktgemisches mit Nitriten beispielsweise Natriumnitrit oder Isoamylnitrit, oder durch die Umsetzung mit α-Hydroxycarbonylverbindungen beispielsweise Hydroxyaceton, Dihydroxyaceton, 2-Hydroxycyclopentanon (Glutaroin), 2-Hydroxycyclohexanon (Adipoin), Glucose sowie anderen reduzierbaren Zucker zu den entsprechenden Glykolen reduziert werden.

Als mögliche Reduktionsmittel können auch Endiole beispielsweise Ascorbinsäure oder Verbindungen, welche eine B-H - Bindung enthalten beispielsweise Natriumborhydrid oder Natriumcyanborhydrid eingesetzt werden.

Bevorzugt wird im Rahmen der vorliegenden Erfindung zur Reduktion von α-Hydroperoxyalkohol-haltigen Produktgemischen ein Verfahren zur katalytischen Hydrierung gewählt.

Demgemäß betrifft die vorliegende Erfindung auch ein Verfahren zur Epoxidierung der oben genannten Art, welches dadurch gekennzeichnet ist, daß das wenigstens eine Reduktionsmittel wenigstens eine Verbindung aufweist, welche zur katalytischen Hydrierung geeignet ist.

Eine derartig geeignete Verbindung stellt beispielsweise Wasserstoff unter Anwesenheit eines geeigneten Hydrierkatalysators dar.

Im Rahmen der vorliegenden Erfindung versteht man somit unter der katalytischen Hydrierung die Umsetzung eines α-Hydroperoxyalkohol-haltigen Produktgemisches mit Wasserstoff in Anwesenheit eines geeigneten Hydrierkatalysators.

Der in Rede stehende Hydrierkatalysator kann sowohl homogen, als auch heterogen sein. Im Rahmen der Erfindung wird die katalytische Hydrierung bevorzugt in Anwesenheit eines heterogenen Katalysators ausgeführt.

Der Hydrierkatalysator weist mindestens ein Aktivmetall aus der VIIb-, der VIII-, der Iasowie der Ib-Nebengruppe des Periodensystems der Elemente, einzeln oder als Gemisch aus zwei oder mehr davon, auf.

Im Rahmen der Erfindung werden hierbei Palladium (Pd), Platin (Pt), Rhodium (Rh), Ruthenium (Ru), Iridium (Ir), Osmium (Os), Eisen (Fe), Cobalt (Co), Nickel (Ni) sowie Kupfer (Cu), bevorzugt Pd, Pt, Rh, Ru und Ir, besonders bevorzugt Pd eingesetzt.

Diese sind auch in Pulverform einsetzbar. Das Aktivmetallpulver kann nach verschiedenen Methoden hergestellt werden. Üblich sind beispielsweise thermische Zersetzung von Aktivmetallsalzen, die Reduktion von wässrigen oder nichtwäßrigen Aktivmetallsalzlösungen mit beispielsweise Hydrazin, Formaldehyd, Wasserstoff oder anderen Reduktionsmitteln. Aktivmetallpulver können mindestens ein Aktivmetall oder auch eine Mischung aus zwei oder mehr davon aufweisen.

Auch Aktivmetallkörper können zur Hydrierung eingesetzt werden. Dabei kommen bevorzugt Folien, Drähte, Netze (herstellbar durch Weben, Stricken und Wirken), Granalien und Kristallitpulver hergestellt aus mindestens einem Aktivmetall oder einer Mischung aus zwei oder mehr davon zur Anwendung.

Weiterhin sind auch Aktivmetalloxide beispielsweise als Suspensionen mindestens eines Aktivmetalls oder einer Mischung aus zwei oder mehr davon zur katalytischen Hydrierung einsetzbar.

Im Rahmen der vorliegenden Erfindung werden bevorzugt Hydrierkatalysatoren eingesetzt, welche einen Verbund aus mindestens einem Aktivmetall oder einer Mischung aus zwei oder mehr davon und mindestens einem Trägermaterial aufweisen.

Der Gehalt an Aktivmetallen befindet sich dabei, wenn es sich um ein Aktivmetall aus der Gruppe bestehend aus Pd, Pt, Rh, Ir, Ru und Os handelt, in der Regel in einem Bereich von 0,01 bis 10 Gew.-%. Ist ein Aktivmetall der Gruppe bestehend aus Fe, Co, Ni, und Cu enthalten, liegt der Gehalt in der Regel in einem Bereich von 1 bis 80 Gew.-%.

Als Trägermaterial können alle dem Fachmann hierfür bekannten Materialien eingesetzt werden, die eine für den jeweiligen Anwendungsbereich geeignete chemische und thermische Stabilität aufweisen. Beispielsweise poröse Oxide wie Aluminiumoxid, Siliziumdioxid, Alumosilkate, Zeolithe, Titanoxid, Zirkonoxid, Chromoxid, Zinkoxid, Magnesiumoxid, Seltenerdoxide, aber auch Aktivkohle oder Mischungen aus zwei oder mehr der eben genannten Verbindungen. Weiterhin können auch alle in EP 0 827 944 A1 beschriebenen Arten von Trägermaterialien eingesetzt werden.

Die aus mindestens einem Aktivmetall oder aus einer Mischung aus zwei oder mehr davon und mindestens einem Träger herstellbaren Verbunde, sogenannte Trägerkatalysatoren können auf jede, dem Fachmann bekannte Art und Weise hergestellt werden.

Beispielsweise sind derartige Trägerkatalysatoren im allgemeinen erhältlich, in dem man den mindestens einen Träger mit einer Lösung des mindestens einen Aktivmetalls oder einer Mischung aus zwei oder mehr davon tränkt, wobei man bei mehreren Aktivmetallen diese gleichzeitig oder nacheinander zugeben kann. Dabei ist es möglich, den Träger ganz oder auch nur teilweise mit einer solchen Lösung zu tränken.

Selbstverständlich ist es auch möglich, die in Rede stehende Lösung von entsprechenden Aktivmetallen nach an sich bekannten Methoden auf den Träger aufzusprühen bzw. diesen damit zu bedampfen oder die Aktivmetalle durch elektrochemische Abscheidung auf diesem aufzubringen. Das Aufbringen des mindestens einen Aktivmetalls oder einer Mischung aus zwei oder mehr davon, kann darüber hinaus auch in einer in EP 0 827 944 A1 beschriebenen Weise erfolgen.

Generell bestimmt sich bei beiden Verfahren die gewünschte Aktivmetallbeladung des Trägers über die jeweils gewählte Konzentration der Aktivmetalllösung.

Die somit hergestellten Katalysatorvorläufer können das mindestens eine Aktivmetall oder die Mischung aus zwei oder mehr davon radial gleichverteilt oder angereichert in einer Schale enthalten. Unter einer Schale versteht man im vorliegenden Fall einen äußeren radialen Bereich des Katalysatorvorläufers (Trägers), der das Aktivmetall in einer - verglichen mit den übrigen bereichen des Katalysatorvorläufers (Trägers) höheren Konzentration aufweist.

Nach dem Tränken oder Sprühen können im allgemeinen weitere Schritte folgen, wie beispielsweise ein Trocknungsschritt und/oder eine Wärmebehandlung sowie ein Calcinierungsschritt.

Trägerkatalysatoren sind im allgemeinen auch erhältlich, in dem man mindestens eine Vorstufe der Aktivmetalle in Gegenwart mindestens eines geeigneten Trägermaterials alkalisch oder reduktiv ausfällt. Die auf diesem Weg erhaltenen Katalysatorvorläufer können dann in eine für die jeweilige Anwendung geeignete Form gebracht werden. Beispielsweise in Strängen oder Tablettenform. Anschließend können im allgemeinen auch die oben angeführten weiteren Schritte wie Trocknung, Wärmebehandlung und Calcinierung erfolgen.

Als Vorstufen der Aktivmetalle kommen grundsätzlich alle wasserlöslichen Aktivmetallverbindungen in Frage. Beispielsweise gut wasserlösliche Salze oder Komplexsalze der Aktivmetalle wie Nitrate, Nitrosylnitrate, Chloride, Acetate, Formiate und Sulfate sowie Chlorometallate.

Das Trocknen der Katalysatorvorläufer kann nach allen dem Fachmann bekannten Trocknungsarten erfolgen. Im Rahmen der vorliegenden Erfindung wird der Trocknungsprozess bevorzugt bei Temperaturen im Bereich von 80 bis 150°C, besonders bevorzugt im Bereich von 80 bis 120°C durchgeführt.

Das Calcinieren der Katalysatorvorläufer kann auf jede dem Fachmann bekannte Weise erfolgen. Im Rahmen der vorliegenden Erfindung setzt man die erhaltenen Katalysatorvorläufer vorzugsweise bei Temperaturen im Bereich von 150 bis 500°C, besonders bevorzugt im Bereich von 200 bis 450°C einem Gastrom, welcher Luft oder Stickstoff aufweist, aus.

In der Regel kann im Anschluß an den Calcinierungsprozess die Aktivierung der so erhaltenen Katalysatorvorläufer erfolgen.

Die Aktivierung kann durch alle dem Fachmann hierfür bekannten Verfahren erfolgen, bei welchen die Katalysatorvorläufer einer reduzierenden Atmosphäre, beispielsweise einer Wasserstoff-haltigen Atmosphäre bei gegebenenfalls erhöhter Temperatur, ausgesetzt werden.

Im Rahmen der Erfindung können Katalysatorvorläufer, welche ein Aktivmetall der Gruppe bestehend aus Pd, Pt, Rh, Ir, Ru und Os enthalten in einem Temperaturbereich von 80 bis 250°C, vorzugsweise von 80 bis 180°C mit Wasserstoff behandelt werden. Katalysatorvorläufer, welche ein Aktivmetall der Gruppe bestehend aus Fe, Co, Ni, und Cu enthalten werden bevorzugt in einem Temperaturbereich von 150 bis 500°C, besonders bevorzugt von 200 bis 450°C mit Wasserstoff behandelt.

Die Dauer der Behandlung mit Wasserstoff bei gegebenenfalls erhöhten Temperaturen richtet sich nach der Konzentration des mindestens einen Aktivmetalls oder der Mischung aus zwei oder mehr davon.

Im Rahmen der Erfindung beträgt die Dauer der Behandlung bei Katalysatorvorläufern, welche ein Aktivmetall der Gruppe bestehend aus Pd, Pt, Rh, Ir, Ru und Os enthalten, vorzugsweise 0,5 bis 24 Stunden, besonders bevorzugt ist eine Dauer von 1 bis 5 Stunden. Bei Katalysatorvorläufem, welche ein Aktivmetall der Gruppe bestehend aus Fe, Co, Ni, und Cu enthalten, beträgt die Dauer der Behandlung vorzugsweise 12 bis 120 Stunden, besonders bevorzugt 24 bis 72 Stunden.

Die Katalysatorbelastung mit Wasserstoff bei der Aktivierung beträgt im Rahmen der Erfindung in der Regel 1 bis 100 1 kg⁻¹ _{Katalysator} h⁻¹ , bevorzugt jedoch 10 bis 50 l kg⁻¹ _{Katalysator} h⁻¹.

Mit Hilfe der auf die eben beschriebene Weise hergestellten Hydrierkatalysatoren ist es möglich, Hydrierungen in jeder dem Fachmann bekannten Weise durchzuführen, beispielsweise in der Flüssigphase, im Festbett oder in Suspension sowie unter Verwendung der Sumpf- oder Rieselfahrweise. Bevorzugt wird die Hydrierung im Rahmen der vorliegenden Erfindung jedoch im Festbett durchgeführt.

Druck- und Temperaturbereiche der Hydrierung werden entsprechend der zu hydrierenden Substanz bzw. dem Substanzgemisch gewählt. Im Rahmen der vorliegenden Erfindung wird die Hydrierung vorzugsweise bei Druckbereichen von 1 bis 100 bar_{abs}, besonders bevorzugt 1 bis 10 bar_{abs} und bei Temperaturen im Bereich von vorzugsweise 0 bis 180 °C, weiter bevorzugt 25 bis 120 °C, insbesondere 40 bis 80° C durchgeführt.

Bei einer Hydrierung im Festbett beträgt die Verweilzeit der Flüssigkeit bezogen auf das Reaktorvolumen 1 Sekunde (s) bis 1 Stunde (h), vorzugsweise 10 s bis 20 Minuten (min), insbesondere 30 s bis 5 min.

Demgemäß wird die zur katalytischen Hydrierung eines α-Hydroperoxyalkohol-haltigen Produktgemisches, welches im Rahmen der erfindungsgemäßen Epoxidierung gebildet wird, geeignete Verbindung ausgewählt aus einer Gruppe bestehend aus heterogenen Katalysatoren, welche als Aktivmetall Ru, Ni, Pd, Pt einzeln oder als Gemisch aus zwei oder mehr davon auf einem geeigneten Trägermaterial aufweisen.

Bevorzugt werden dabei solcher Trägerkatalysatoren eingesetzt, die nach einem der oben beschriebenen Verfahren hergestellt und zur Hydrierung des α-Hydroperoxyalkoholhaltigen Produktgemisches eingesetzt werden.

Die Hydrierung des α-Hydroperoxyalkohol-haltigen Produktgemisches kann innerhalb der verschiedenen Verfahrensschritte, welche die erfindungsgemäße Epoxidierung aufweist, durchgeführt werden.

Demgemäß betrifft die vorliegende Erfindung auch ein Verfahren zur Epoxidierung einer organischen Verbindung mit wenigstens einer C-C-Doppelbindung mit Wasserstoffperoxid in Gegenwart wenigstens einer katalytisch aktiven Verbindung und wenigstens eines Lösungsmittels, dadurch gekennzeichnet, daß es wenigstens die folgenden Schritte umfaßt:
(i) eine organische Verbindung mit wenigstens einer C-C-Doppelbindung wird in Gegenwart wenigstens einer katalytisch aktiven Verbindung und wenigstens eines Lösungsmittels mit Wasserstoffperoxid zu einem Produktgemisch P 1 aufweisend α-Hydroperoxyalkohole umgesetzt;
(ii) aus P1 bzw. P1' wird ein bei der Umsetzung in Schritt (i) entstandenes Epoxid sowie die nicht umgesetzte organische Verbindung mit wenigstens einer C-C-Doppelbindung abgetrennt, wobei ein Produktgemisch P2 bzw. P2' erhalten wird, wobei P2 die bei der Umsetzung in Schritt (i) als Nebenprodukt entstehenden α-Hydroperoxyalkohole aufweist, P2' weitgehend frei von α-Hydroperoxyalkoholen ist.
(iii) P1 und/oder P2 werden mit wenigstens einem Reduktionsmittel versetzt, wobei die α-Hydroperoxyalkohole zu entsprechenden Glykolen unter Erhalt eines Produktgemisches P1' bzw. P3 umgesetzt werden.

Die Umsetzung in Schritt (i) kann in einem Reaktor, vorzugsweise einem annähernd isothermen Rohrbündelreaktor, in welchem unter Druck und ohne Gasphase gearbeitet wird, durchgeführt werden.

Das sich dabei bildende Produktgemisch P 1 weist in der Regel neben dem Nebenprodukt α-Hydroperoxyalkohol, das eingesetzte Lösungsmittel, Wasser, das gewünschte Epoxid, Anteile an nicht umgesetzter organischer Verbindung mit wenigstens einer C-C-Doppelbindung sowie weitere Nebenprodukte, wie beispielsweise Sauerstoff sowie Produkte, welche sich durch die Addition des Lösungsmittels oder des Wassers an das Epoxid bilden, auf.

Bereits nach diesem Verfahrensschritt (i) ist es möglich, das α-Hydroperoxyalkoholhaltige Produktgemisch P 1 unter Durchführung von Schritt (iii) in der oben beschriebenen Art zu reduzieren, wobei die α-Hydroperoxyalkohole zu den entsprechenden Glykolen unter Erhalt eines weiteren Produktgemisches P1' umgesetzt werden.

Dieser Schritt kann aber auch unterbleiben.

Führt man Schritt (iii) unmittelbar im Anschluß an Schritt (i) durch, so ist das Produktgemisch P1', welches in Schritt (ii) eingesetzt wird weitgehend frei von α-Hydroperoxyalkoholen.

Wird der Schritt (iii) dem Schritt (i) nicht unmittelbar angeschlossen, so enthält P1 noch α-Hydroperoxyalkohole.

Aus Produktgemisch P1 bzw. P1' kann in Schritt (ii) das gewünschte Epoxid, als auch die nicht umgesetzte organische Verbindung mit wenigstens einer C-C-Doppelbindung abgetrennt werden. Diese Abtrennung erfolgt in Schritt (ii) unter Erhalt eines Produktgemisches P2 bzw. P2'.

Die Abtrennung kann durch jedes dem Fachmann für diesen Zweck bekannte Verfahren erfolgen. Beispielsweise durch Destillation, geeignete Fällungsreaktionen, Extraktion und auch Membranpermeation.

Im Rahmen der vorliegenden Erfindung wird die Abtrennung vorzugsweise destillativ durchgeführt.

Demgemäß betrifft die vorliegende Erfindung weiterhin ein Verfahren der erfindungsgemäßen Art, welches dadurch gekennzeichnet ist, daß die Abtrennung des in Schritt (i) anfallenden Epoxids sowie der nicht umgesetzten organischen Verbindung mit wenigstens einer C-C-Doppelbindung in Schritt (ii) destillativ erfolgt.

Die destillative Abtrennung kann mit einer dem Fachmann für diesen Zweck bekannten Destillationsvorrichtungen erfolgen. Sie kann sowohl kontinuierlich, als auch diskontinuierlich durchgeführt werden.

An die Durchführung der Destillation von α-Hydroperoxyalkohol-freien Produktgemischen wie P1' sind keine besonderen Bedingungen geknüpft. Sie kann unter den allgemeinen, dem Fachmann gängigen Destillationsbedingungen bezüglich der Wahl des Drucks, der Temperatur sowie der Verweildauer im Destillationssumpf durchgeführt werden.

Erfolgt jedoch die Destillation von noch α-Hydroperoxyalkohol-haltigen Produktgemischen wie P1, so ist dabei auf die Einhaltung von bestimmten Bedingungen zu achten. Die Destillation wird auf eine Weise durchgeführt, bei welcher gewährleistet werden kann, daß α-Hydroperoxyalkohole weitgehend, d.h. bevorzugt zu > 80 %, besonders bevorzugt > 90 %, sowie vorzugsweise vollständig unzersetzt bleiben.

Demgemäß betrifft die vorliegende Erfindung auch ein Verfahren der erfindungsgemäßen Art, welches dadurch gekennzeichnet ist, daß die destillative Abtrennung des im Schritt (i) anfallenden Epoxids sowie der nicht umgesetzten organischen Verbindung mit wenigstens einer C-C-Doppelbindung in Schritt (ii) bei Temperaturen unter 80 °C gemessen im Destillationssumpf der zur Destillation verwendeten Vorrichtung, sowie mit einer Verweildauer von unter 4 Stunden in diesem durchgeführt wird.

Vorzugsweise wird mit eine Verweildauer von unter 2 Stunden, besonders bevorzugt von unter 1 Stunde, sowie bei Temperaturen bevorzugt im Bereich unter 75°C, besonders bevorzugt unter 70°C im Destillationssumpf gearbeitet.

Im Allgemeinen arbeitet man bei einer Temperatur, welche sich bei der Destillation unter Normaldruck einstellt.

Besonders bevorzugt wird bei 65 °C ± 2 °C im Destillationssumpf und einer Verweildauer von ca. 2 h gearbeitet.

Neben dem gewünschten Epoxid sowie der nicht umgesetzten organischen Verbindung mit wenigstens einer C-C-Doppelbindung werden auch gegebenenfalls in P1 bzw. P1' vorhandener Sauerstoff, andere leichtsiedende Nebenprodukte sowie ein geringer Anteil an Lösungsmittel in einer Destillationskolonne über Kopf abgetrennt. Den Sumpf dieser Kolonne bildet das Produktgemisch P2. P2 besteht hauptsächlich aus Lösungsmittel, Wasser, nicht umgesetztem Wasserstoffperoxid und hochsiedenden sowie anderen Nebenprodukten. P2 weist noch α-Hydroperoxyalkohole auf.

Das α-Hydroperoxyalkohol-haltige Produktgemisch P2 kann unter Durchführung der in Schritt (iii) beschriebenen Umsetzung zu einem weiteren Produktgemisch P3 reduziert werden, wobei die α-Hydroperoxyalkohole dabei zu den entsprechenden Glykolen umgesetzt werden.

Aus wirtschaftlichen Gründen ist man bestrebt das Lösungsmittel aus dem Produktaustrag zurückzugewinnen, es zu reinigen und dem Verfahren wieder zuzuführen. Um dabei die eingangs beschriebenen Probleme, welche sich bei der Rückgewinnung in Anwesenheit von α-Hydroperoxyalkoholen ergeben, zu vermeiden, wird das Lösungsmittel in der Regel aus dem Produktgemisch zurückgewonnen, welches bereits reduziert wurde, d.h., welches weitgehend frei von α-Hydroperoxyalkoholen ist.

Das Produktgemisch P2 ist noch α-Hydroperoxyalkohol-haltig. Demgemäß ist es vorteilhaft dieses zunächst wie oben beschrieben zum Produktgemisch P3 zu reduziert, um im Anschluß daran aus P3 das Lösungsmittel abtrennen, reinigt und dem Verfahren wieder zuführen zu können.

Das Produktgemisch P2' ist bereits α-Hydroperoxyalkohol-frei. Somit kann aus ihm direkt das Lösungsmittel abgetrennt und zur Wiedereinsetzung aufbereitet werden.

Demgemäß betrifft die vorliegende Erfindung auch ein Verfahren der erfindungsgemäßen Art, welches dadurch gekennzeichnet ist, daß im Anschluß an Schritt (iii) das im dann jeweiligen Produktgemisch enthaltene Lösungsmittel ganz oder teilweise abgetrennt und gegebenenfalls in Schritt (i) zurückgeführt wird. Vorzugsweise wird das Lösungsmittel zurückgeführt.

Weiterhin vorteilhaft bei der Reduktion α-Hydroperoxyalkohol-haltiger Produktgemische ist, daß neben den α-Hydroperoxyalkoholen die sich gegebenenfalls noch im Produktgemisch befindliche Restmenge an Wasserstoffperoxid zu Wasser reduziert wird. Dadurch verringert sich auch die Gefahr, welche sich aus der unkontrollierten Zersetzung von Wasserstoffperoxid unter Bildung von Sauerstoff ergeben kann.

Dies macht sich besonders vorteilhaft bemerkbar, wenn die Reduktion von Schritt (iii) erst im Anschluß an die Abtrennung von Schritt (ii) erfolgt.

Sowohl wirtschaftlich als auch umweltpolitisch ist es erstrebenswert, die bei dem Verfahren zur Epoxidierung anfallenden Nebenprodukte sinnvoll zu nutzen. So ist es bei dem vorliegenden erfindungsgemäßen Verfahren möglich, die durch die Reduktion des α-Hydroperoxyalkohol-haltigen Produktgemisches entstehenden Glykole (1,2-Diole) als weitere Wertprodukte abzutrennen und anderen Verfahren beispielsweise als Edukte oder Lösungsmittel zuzuführen.

Demgemäß betrifft die vorliegende Erfindung auch ein Verfahren der erfindungsgemäßen Art, welches dadurch gekennzeichnet ist, daß die bei einer Reduktion eines α-Hydroperoxyalkohol-haltigen Produktgemisches anfallenden Glykole als weitere Wertprodukte abgetrennt werden können.

Die Abtrennung der Glykole kann durch jedes dem Fachmann hierfür bekannte Verfahren erfolgen, beispielsweise Destillation, Extraktion oder Membranpermeation.

Die abgetrennten Glykole sind vielseitig einsetzbar. Beispielsweise als Edukte für Synthesen im Bereich der Kunststoffindustrie oder auch im Rahmen von Naturstoffsynthesen sowie allgemein als Lösungsmittel in weiten Bereichen der Großindustrie.

Demgemäß betrifft die vorliegende Erfindung auch die Verwendung der als weitere Wertprodukte aus dem erfindungsgemäßen Verfahren abgetrennten Glykole zur weiteren Verwendung in allen dem Fachmann bekannten Einsatzgebieten.

Vorzugsweise wird das erfindungsgemäße Verfahren, zur Epoxidierung von Propen zu Propenoxid mit Wasserstoffperoxid in methanolischer Lösung in Gegenwart eines Titansilikalits mit MFI-Struktur eingesetzt. Diese Ausführungsform des erfindungsgemäßen Verfahrens soll nachfolgend nochmals detailliert beschrieben werden.

Die bei dieser Reaktion als Nebenprodukte entstehenden α-Hydroperoxyalkohole sind 1-Hydroperoxypropanol-2 sowie 2-Hydroperoxypropanol-1. Durch die erfindungsgemäße Reduktion können sie zu Propylenglykol (1,2-Propandiol) reduziert werden, welches als weiteres Wertprodukt abgetrennt werden kann.

Propylenglykol ist vielseitig einsetzbar. Beispielsweise als Frostschutzmittel, als Bremsflüssigkeit, zur Herstellung von Alkyl- und Polyesterharzen, als Weichmacher für Vinylharze, als Lösungsmittel für Fette, Öle, Harze, Wachse, Farbstoffe usw. Auch in der Nahrungsmittelindustrie wird Propylenglykol beispielsweise als Lösungsmittel für Farbstoffe und Aromen eingesetzt. Weiterhin findet es Anwendung als Feuchthaltemittel für Tabakprodukte und in Kosmetika, sowie als Tränksubstanz bei verschiedenen Salben, Cremes und Arzneimitteln. Trennt man die optisch aktive Form ab, so kann diese als chiraler Baustein in verschiedenen organischen Synthesen eingesetzt werden.

Propylenglykol ist auch ganz allgemeinen eine Verbindung, welche sehr häufig als Edukt in der Synthese weiterer Ausgangsprodukte für die chemische Industrie verwendet. So wird es nach der Veresterung oder Veretherung einer oder beider Hydroxylgruppen häufig als Lösungsmittel, als Weichmacher oder Verdickungsmittel sowie als Emulgator eingesetzt. Auch die Polyadditon von Propylenglykol führt zu weiteren wichtigen Polymeren, einsetzbar in industriellen Verfahren.

In einer bevorzugten Ausführungsform des erfindungsgemäßen Verfahrens zur Epoxidierung von Propen zu Propenoxid wird Propen mit Wasserstoffperoxid in Methanol an einem verstrangten TS-1-Katalysator im Hauptreaktor (annähernd isothermer Rohrbündelreaktor unter Druck, ohne Gasphase) umgesetzt (Schritt (i); führt zu einem Produktgemisch P1). Dabei werden zwischen 75 und 95 % des Wasserstoffperoxids umgesetzt.

Nach dem Hauptreaktor wird das gebildete Propylenoxid zusammen mit nicht umgesetztem Propen, Sauerstoff sowie weiteren leichtsiedenden Nebenprodukten und einem geringen Anteil an Methanol in einer Destillationskolonne über Kopf abgetrennt (Schritt (ii); führt zu einem Produktgemisch P2, dem Destillationssumpf).

Der Sumpf dieser Kolonne (P2) besteht hauptsächlich aus Methanol, Wasser und dem nicht umgesetzten Wasserstoffperoxid sowie hochsiedenden Nebenprodukten. Dieser wird in mindestens einen weiteren Reaktor, einen sogenannten Nachreaktor, überführt. Vorzugsweise handelt es sich bei dem Nachreaktor um einen annähernd adiabatischen Rohrreaktor, in welchem unter Druck und ohne Gasphase gearbeitet wird. Dort wird der Sumpf (P2) erneut mit Propen umgesetzt (entspricht der erneuten Durchführung eines Schritt (i)).

Bei diesem Schritt entsteht ein weiteres Produktgemisch (P1), welches hauptsächlich aus Methanol, Wasser, Propylenoxid und Nebenprodukten, u.a. auch die α-Hydroperoxyalkohole wie 1-Hydroperoxypropanol-2 sowie 2-Hydroperoxypropanol-1 besteht. Weiterhin enthält dieses Produktgemisch in der Regel weniger als 0,1 Gew.-% unumgesetztes Wasserstoffperoxid.

Nach der Umsetzung im Nachreaktor wird der daraus resultierende Produktgemisch zur Gewinnung des Propylenoxids und gegebenenfalls des Propylenglykols weiter aufgearbeitet.

Dabei besteht zum einen die Möglichkeit das Produktgemisch zunächst zu reduzieren (Schritt (iii)), um die zu Problemen führenden Nebenprodukte (α-Hydroperoxyalkohole) zu Propylenglykol umzusetzen. Im Anschluß daran kann Propylenoxid sowie nicht umgesetztes Propen destillativ (Schritt (ii)) zur weiteren Aufarbeitung abgetrennt werden. Dann folgt die Rückgewinnung des Lösungsmittels Methanol sowie die Abtrennung des weiteren Wertprodukts Propylenglykol aus dem verbleibenden Destillationssumpf.

Zum anderen besteht die Möglichkeit aus dem Produktgemisch des Nachreaktors zunächst Propylenoxid sowie nicht umgesetztes Propen destillativ unter Einhaltung der oben geschilderten Reaktionsbedingungen bei Destillation von α-Hydroperoxyalkohol-haltigen Produktgemischen, zu entfernen (Schritt (ii)), um im Anschluß daran den Destillationssumpf zu reduzieren (Schritt (iii)) und aus ihm sowohl das dabei entstehende Propylenglykol als auch das Lösungsmittel Methanol abzutrennen.

Weiterhin ist es auch möglich die Produktgemische welche nach Durchführung von Schritt (iii) erhalten werden und welche weitgehend α-Hydroperoxyalkohol-frei sind, zu vereinen. Aus diesem können dann ebenfalls Propylenglykol und Methanol abgetrennt werden.

Selbstverständlich kann jeder durchzuführende Schritt mehrfach hintereinander erfolgen, wobei das jeweils dabei entstehenden Produktgemisch gesammelt wird und die gesammelten Produktgemische dann gemeinsam dem sich jeweils als nächstes anschließenden Schritt zugeführt werden.

Ist im Produktaustrag des Nachreaktors noch mehr als 0,1 Gew.-% Wasserstoffperoxid feststellbar, so kann selbstverständlich der bei der Destillation (Schritt (ii)) anfallende Sumpf nochmals in einem weiteren Nachreaktor in der oben beschriebenen Schrittfolge erneut mit Propen umgesetzt werden und so eine Reaktorkaskade verwirklicht werden.

Im Rahmen der Erfindung wird die folgenden Reaktionsabfolge (A) bevorzugt:
1. Eine organische Verbindung mit wenigstens einer C-C-Doppelbindung wird in Gegenwart wenigstens einer katalytisch aktiven Verbindung und wenigstens eines Lösungsmittels mit Wasserstoffperoxid in einem Schritt (i) zu einem Produktgemisch P1 (α-Hydroperoayalkohol-haltig) umgesetzt;
2. Produktgemisch P1 wird in einem Schritt (iii) mit wenigstens einem Reduktionsmittel versetzt, wobei die α-Hydroperoxyalkohole zu den entsprechenden Glykolen unter Erhalt eines weiteren Produktgemisches P1' (α-Hydroperoxyalkohol-frei) umgesetzt werden;
3. Aus P1'wird ein bei der Umsetzung in 1. (Schritt (i)) entstandenes Epoxid sowie die nicht umgesetzte organische Verbindung mit wenigstens einer C-C-Doppelbindung in Schritt ii) abgetrennt, wobei ein Produktgemisch P2' erhalten wird;
4. Aus P 2' können jetzt die den α-Hydroperoxyalkoholen entsprechenden Glykole sowie das Lösungsmittel auf die oben beschriebne Art abgetrennt werden.

Besonders bevorzugt wird jedoch die folgende Reaktionsabfolge (B):
1. Eine organische Verbindung mit wenigstens einer C-C-Doppelbindung wird in Gegenwart wenigstens einer katalytisch aktiven Verbindung und wenigstens eines Lösungsmittels mit Wasserstoffperoxid in einem Schritt (i) zu einem Produktgemisch P 1 (α-Hydroperoxyalkohol-haltig) umgesetzt;
2. Aus P 1 wird ein bei der Umsetzung in 1. (Schritt (i)) entstandenes Epoxid sowie die nicht umgesetzte organische Verbindung mit wenigstens einer C-C-Doppelbindung in Schritt (ii) abgetrennt, wobei ein Produktgemisch P2 erhalten wird;
3. Produktgemisch P2 wird in einem Schritt (iii) mit wenigstens einem Reduktionsmittel versetzt, wobei die α-Hydroperoxyalkohole zu den entsprechenden Glykolen unter Erhalt eines weiteren Produktgemisches P3 (α-Hydroperoxyalkohol-frei) umgesetzt werden;
4. Aus P3 können jetzt die den α-Hydroperoxyalkoholen entsprechenden Glykole sowie das Lösungsmittel auf die oben beschriebne Art abgetrennt werden.

Die Erfindung soll nunmehr anhand von Beispielen näher erläutert werden.

### Beispiel (B1)

### Umsetzung von Propen mit Wasserstoffperoxid

In einen Rohrreaktor (Länge: 2 m, Durchmesser: 45 mm) mit Kühlmantel und Druckregelung wurden 620 g eines verstrangten TS-1-Katalysators (hergestellt nach WO 98/55229) eingefüllt.

Durch diesen Reaktor wurde ein Gemisch aus Methanol (1560 g/h), wäßriger Wasserstoffperoxid-Lösung (330 g/h, ca. 40 Gew.-% in Wasser) und Propylen (245 g/h) bei einem Druck von 20 bar geleitet.

Die Temperatur des Kühlmediums im Reaktormantel wurde so eingestellt (je nach Katalysatoraktivität zwischen 20 und 50°C), daß im Reaktoraustrag annähernd 90% des Wasserstoffperoxids umgesetzt waren. Der Umsatz wurde durch Messung der Konzentration von Wasserstoffperoxid im Austrag mit der Titanylsulfat-Methode bestimmt.

Der Reaktoraustrag wurde entspannt und in einer kontinuierlichen Destillationskolonne bei Normaldruck aufgearbeitet. Die Destillationsbedingungen wurden so gewählt, dass praktisch die gesamte Menge an nicht umgesetztem Propylen und an gebildetem Propylenoxid über Kopf abdestilliert wurde. Ein gewisser Anteil des Methanols geht mit über Kopf (ungefähr die gleiche Menge wie Propylenoxid). Die Temperatur im Sumpf der Kolonne betrug ca. 67°C und die Verweilzeit im Sumpf lag bei ca. 1 Stunde.

Im Sumpf wurde ein Gemisch mit folgender durchschnittlicher Zusammensetzung (in Gew.-%) gewonnen:
Methanol (81), Wasser (17), Wasserstoffperoxid (0.9), Hydroperoxypropanole (Summe von beiden Isomeren, 0.4), 1-Methoxypropanol-2 (0.3), 2-Methoxypropanol-1 (0.2), Propylenglykol (0.1 ).

### Vergleichsbeispiel (V1)

Der Reaktoraustrag aus B1 wurde ohne weitere Behandlung in eine zweite kontinuierliche Destillationskolonne eingespeist, welche bei Normaldruck betrieben wurde.

In dieser Kolonne wurde das Gemisch in eine weitgehend wasserfreie Methanolfraktion (Kopfprodukt) und eine weitgehend methanolfreie Wasserfraktion (Sumpfprodukt) getrennt. Die Sumpftemperatur betrug ca. 99°C und die Verweilzeit im Sumpf lag bei ca. 1 Stunde.

Wegen der höheren Temperatur in dieser Kolonne zersetzten sich darin sowohl Wasserstoffperoxid als auch die α-Hydroperoxypropanole.

Die Methanolfraktion am Kopf enthielt folgende Verunreinigungen (Gew.-%):
Acetaldehyd (0.1), 1,1-Dimethoxyethan (0.2), Methylformiat (0.002).

Außerdem enthielt das nicht kondensierbare Abgas am Kopf der Kolonne erhebliche Sauerstoffmengen und muß mit Stickstoff inertisiert werden. Dieser Methanolstrom kann somit nicht ohne weitere Behandlungen für die Propylen-Epoxidierung wieder eingesetzt werden.

Weder Acetaldehyd noch 1,1-Dimethoxyethan noch Methylformiat wurden in den Epoxidierungssreaktoren abgebaut. Acetaldehyd und 1,1-Dimethoxyethan würden sich somit im weiteren Verlauf im Methanolstrom aufpegeln. Das Methylformiat dagegen würde sich nicht aufpegeln, sondern (wegen der sehr ähnlichen Siedepunkte) bei Propylenoxid als schwer abtrennbare Verunreinigung auftreten.

Die Wasserfraktion am Sumpf der Kolonne enthielt folgende Verunreinigungen (Gew.%):
1-Methoxypropanol-2 (1.5), 2-Methoxypropanol-1 (1.3), Propylenglykol (0.9), Ameisensäure (0.5), Formaldehyd (0.2) und Hydroxyaceton (0.2). Obwohl Propylenglykol als potentielles Wertprodukt anzusehen ist, ist die Konzentration zu gering, um es wirtschaftlich aus diesem Strom zurückzugewinnen.

### Beispiel (B2)

Das Gemisch aus B 1 wurde gesammelt und ca. 5 kg davon wurden in einem Autoklav bei 50°C und 10 bar Wasserstoff solange hydriert bis kein Wasserstoff mehr aufgenommen wurde (ca. 1Stunde). Als Hydrierkatalysator wurde ein Trägerkatalysator mit 5 Gew.-% Pd auf Aktivkohle eingesetzt (10 g).

Der Austrag aus der Hydrierung enthielt keine Peroxide und wurde nach Entfernung des Katalysators durch Filtration in einer diskontinuierlichen Destillation bei Normaldruck in eine weitgehend wasserfreie Methanolfraktion (Kopfprodukt) und eine weitgehend methanolfreie Wasserfraktion (Sumpfprodukt) getrennt. Die Sumpftemperatur betrug am Ende der Destillation ca. 99°C und die Destillationszeit betrug ca. 8 Stunden.

Die Methanolfraktion am Kopf enthielt außer Wasser (ca. 100 ppm) keine anderen nachweisbaren Verunreinigungen. Dieser Methanolstrom konnte somit ohne weitere Behandlungen erneut in das Verfahren zur Epoxidierung von Propylen zurückgeführt werden.

Die im Sumpf verbleibende Wasserfraktion enthielt nur folgende Verunreinigungen (Gew.-%):
1-Methoxypropanol-2 (1.5), 2-Methoxypropanol-1 (1.2), Propylenglykol (2.5) und Dipropylenglykolmonomethylether (Isomerengemisch, in Spuren).

Weder Ameisensäure noch Formaldehyd noch Hydroxyaceton konnten nachgewiesen werden.

Durch die wesentlich höhere Konzentration an Propylenglykol in der Wasserfraktion kann dieses als Wertprodukt daraus isoliert und weiter wirtschaftlich verwertet werden.

Somit zeigen die Beispiele, daß das erfindungsgemäße Verfahren die Nachteile, welche im Stand der Technik, verursacht durch die als Nebenprodukte entstehenden α-Hydroperoxyalkohole auftreten, vermeidet. Es stellt somit ein technisch hochwertiges Verfahren zur Epoxidierung von Verbindungen mit wenigstens einer C-C-Doppelbindung dar, bei welchem durch die erfindungsgemäße Verfahrensführung mit einem gegenüber dem Stand der Technik verringerten apparativen Aufwand sowohl das Epoxid in seiner Reinform, als auch weitere Wertprodukte (die den α-Hydroperoxyalkoholen entsprechenden Glykole) unter gleichzeitiger vereinfachter Rückgewinnung des eingesetzten Lösungsmittels abgetrennt werden können.

## Patentansprüche

1. Verfahren zur Epoxidierung einer organischen Verbindung mit wenigstens einer C-C-Doppelbindung mit Wasserstoffperoxid in Gegenwart wenigstens einer katalytisch aktiven Verbindung und wenigstens eines Lösungsmittels, **dadurch gekennzeichnet, daß** es wenigstens die folgenden Schritte umfaßt:
(i) eine organische Verbindung mit wenigstens einer C-C-Doppelbindung wird in Gegenwart wenigstens einer katalytisch aktiven Verbindung und wenigstens eines Lösungsmittels mit Wasserstoffperoxid zu einem Produktgemisch P1 aufweisend α-Hydroperoxyalkohole umgesetzt;
(ii) aus P1 wird ein bei der Umsetzung in Schritt (i) entstandenes Epoxid sowie die nicht umgesetzte organische Verbindung mit wenigstens einer C-C-Doppelbindung abgetrennt, wobei ein Produktgemisch P2 erhalten wird, wobei P2 die bei der Umsetzung in Schritt (i) als Nebenprodukt entstehenden α-Hydroperoxyalkohole aufweist;
(iii) P2 wird mit wenigstens einem Reduktionsmittel versetzt, wobei die α-Hydroperoxyalkohole zu entsprechenden Glykolen unter Erhalt eines Produktgemisches P3 umgesetzt werden,
wobei die Abtrennung des in Schritt (i) anfallenden Epoxids sowie der nicht umgesetzten organischen Verbindung mit wenigstens einer C-C-Doppelbindung in Schritt (ii) destillativ erfolgt und bei Temperaturen unter 80 °C, gemessen im Destillationssumpf der zur Destillation verwendeten Vorrichtung, sowie mit einer Verweildauer von unter 4 Stunden in dieser durchgeführt wird.

2. Verfahren nach Anspruch 1, **dadurch gekennzeichnet, daß** im Anschluß an Schritt (iii) das im Produktgemisch P3 enthaltene Lösungsmittel ganz oder teilweise abgetrennt und gegebenenfalls in Schritt (i) zurückgeführt wird.

3. Verfahren nach Anspruch 1 oder 2, **dadurch gekennzeichnet, daß** das wenigstens eine Reduktionsmittel wenigstens eine Verbindung aufweist, welche zur katalytischen Hydrierung geeignet ist.

4. Verfahren nach Anspruch 3, **dadurch gekennzeichnet, daß** die zur katalytischen Hydrierung geeignete Verbindung ausgewählt wird aus einer Gruppe bestehend aus heterogenen Katalysatoren, welche als Aktivmetall Ru, Ni, Pd, Pt, einzeln oder als Gemisch aus zwei oder mehr davon auf einem geeigneten Trägermaterial aufweisen.

5. Verfahren nach einem der Ansprüche 1 bis 4, **dadurch gekennzeichnet, daß** die bei einer Reduktion eines α-Hydroperoxyalkohol-haltigen Produktgemisches anfallenden Glykole als weitere Wertprodukte abgetrennt werden.

6. Verfahren nach einem der Ansprüche 1 bis 5, **dadurch gekennzeichnet, daß** Propen zu Propenoxid mit Wasserstoffperoxid in methanolischer Lösung in Gegenwart eines Titansilikalits mit MFI-Struktur epoxidiert wird.

## Claims

1. A process for the epoxidation of an organic compound having at least one C-C double bond by means of hydrogen peroxide in the presence of at least one catalytically active compound and at least one solvent, which comprises at least the following steps:
(i) reacting an organic compound having at least one C-C double bond with hydrogen peroxide in the presence of at least one catalytically active compound and at least one solvent to give a product mixture P1 comprising α-hydroperoxyalcohols;
(ii) separating an epoxide formed in the reaction in step (i) and the unreacted organic compound having at least one C-C double bond from P1 to give a product mixture P2, where P2 comprises the α-hydroperoxyalcohols formed as by-product in the reaction in step (i);
(iii) treating P2 with at least one reducing agent so that the α-hydroperoxyalcohols are converted into corresponding glycols to give a product mixture P3
where the epoxide formed in step (i) and the unreacted organic compound having at least one C-C double bond are separated off in step (ii) by distillation and at below 80°C, measured at the bottom of the apparatus used for the distillation, and at a residence time of less than 4 hours in the bottom of the distillation apparatus.

2. The process according to claim 1, wherein the solvent comprised in the product mixture P3 after step (iii) is wholly or partly separated off and, if appropriate, recirculated to step (i).

3. The process according to claim 1 or 2, wherein the reducing agent or agents comprise(s) at least one compound which is suitable for catalytic hydrogenation.

4. The process according to claim 3, wherein the catalyst which is suitable for catalytic hydrogenation is selected from the group consisting of heterogeneous catalysts comprising Ru, Ni, Pd, Pt, either individually or as a mixture of two or more thereof, as active metal on a suitable support material.

5. The process according to any of claims 1 to 4, wherein the glycols formed in a reduction of an α-hydroperoxyalcohol-containing product mixture are separated off as further useful products.

6. The process according to any of claims 1 to 5, wherein propene is epoxidized to propene oxide by means of hydrogen peroxide in methanolic solution in the presence of a titanium silicalite having an MFI structure.

## Revendications

1. Procédé d'époxydation d'un composé organique comportant au moins une double liaison C-C avec du peroxyde d'hydrogène, en présence d'au moins un composé catalytiquement actif et d'au moins un solvant, **caractérisé en ce qu'**il comprend au moins les étapes suivantes :
(i)un composé organique comportant au moins une double liaison C-C est, en présence d'au moins un composé catalytiquement actif et d'au moins un solvant, mis à réagir avec du peroxyde d'hydrogène pour former un mélange produit P1 comportant des α-hydroperoxyalcools,
(ii) on isole à partir de P1 un époxyde formé au cours de la réaction dans l'étape (i) ainsi que le composé organique comportant au moins une double liaison C-C n'ayant pas réagi, en obtenant un mélange produit P2, P2 comportant les α-hydroperoxyalcools formés au cours de la réaction dans l'étape (i) comme sous-produit,
(iii)on ajoute à P2 au moins un réducteur, les α-hydroperoxyalcools étant convertis en glycols correspondants avec obtention d'un mélange produit P3,
la séparation de l'époxyde obtenu dans l'étape (i) ainsi que du composé organique comportant au moins une double liaison C-C n'ayant pas réagi ayant lieu dans l'étape (ii) par distillation et étant effectuée à des températures inférieures à 80°C, mesurées dans le fond de cuve du dispositif utilisé pour la distillation, ainsi qu'avec une durée de séjour de moins de 4 heures dans celui-ci.

2. Procédé suivant la revendication 1, **caractérisé en ce que**, à la suite de l'étape (iii), le solvant contenu dans le mélange produit P3 est totalement ou partiellement isolé et éventuellement recyclé dans l'étape (i).

3. Procédé suivant la revendication 1 ou 2, **caractérisé en ce que** ledit au moins un réducteur présente au moins un composé qui est approprié pour une hydrogénation catalytique.

4. Procédé suivant la revendication 3, **caractérisé en ce que** le composé approprié pour une hydrogénation catalytique est choisi parmi un groupe constitué de catalyseurs hétérogènes, qui, comme métal actif, présentent sur une matière de support appropriée Ru, Ni, Pd, Pt, isolément ou sous la forme d'un mélange de deux ou de plusieurs d'entre eux.

5. Procédé suivant l'une des revendications 1 à 4, **caractérisé en ce que** les glycols obtenus lors d'une réduction d'un mélange produit contenant de l'α-hydroperoxyalcool sont isolés comme autres produits de valeur.

6. Procédé suivant l'une des revendications 1 à 5, **caractérisé en ce qu'**on époxyde du propène en oxyde de propène avec du peroxyde d'hydrogène en solution méthanolique, en présence de silicalite de titane ayant une structure MFI.
